# EUROPÄISCHE PATENTSCHRIFT

(11) **EP 4 094 797 B1**
(45) Veröffentlichungstag und Bekanntmachung des Hinweises auf die Patenterteilung: **05.04.2023**
(21) Anmeldenummer: 21176434.5
(22) Anmeldetag: 28.05.2021
(51) Int. Cl.: A61M 25/00, A61M 39/22

(54) **VORRICHTUNG ZUR APPLIKATION EINES PHARMAZEUTISCHEN FLUIDS**
DEVICE FOR APPLICATION OF A PHARMACEUTICAL FLUID
DISPOSITIF D'APPLICATION D'UN FLUIDE PHARMACEUTIQUE

(43) Veröffentlichungstag der Anmeldung: 30.11.2022
(73) Patentinhaber: Heraeus Medical GmbH, 61273 Wehrheim (DE)
(72) Erfinder: VOGT, Sebastian, 63450 Hanau (DE); KLUGE, Thomas, 63450 Hanau (DE)
(74) Vertreter: Heraeus IP

(56) Entgegenhaltungen:
- EP-A2- 0 191 154
- WO-A1-97/37714
- DE-A1-102013 110 989
- DE-U1- 9 209 164
- FR-A1- 2 707 505

## Beschreibung

Die Erfindung betrifft eine Vorrichtung zur Applikation eines pharmazeutischen Fluids umfassend einen Schlauch und ein Ventilelement, wobei der Schlauch über ein erstes Schlauchende mit einem Reservoir für das pharmazeutische Fluid fluidleitend verbindbar ist und wobei das Ventilelement in einem zweiten Schlauchende des Schlauchs angeordnet ist.

Die Erfindung betrifft weiterhin ein System zum Applizieren eines pharmazeutischen Fluids umfassend ein Reservoir für das pharmazeutische Fluid und eine derartige Vorrichtung und ein Verfahren zur Applikation eines pharmazeutischen Fluids mittels einer derartigen Vorrichtung.

Gegenstand der Erfindung ist insbesondere eine medizinische Vorrichtung zur temporären, lokalen Applikation von pharmazeutischen Fluiden oder anderen medizinischen Fluiden über einen Zeitraum von Stunden bis zu mehreren Tagen.

### Hintergrund der Erfindung

Die lokale Applikation von pharmazeutischen Fluid, wie beispielsweise von mit Antibiotika versetzten Lösungen, ist schon seit Jahrzehnten bekannt und bewährt sich insbesondere bei der Behandlung beziehungsweise der Beruhigung von Infektionen des Knochengewebes. Dabei kann man in nicht-resorbierbare und in resorbierbare beziehungsweise biodegradierbare Wirkstoffträger unterscheiden. Das Einleiten von Fluiden in Hohlräume zum Zweck der Spülung und Desinfektion kann aber auch zur Desinfektion und Reinigung von medizinischen Implantaten und Geräten mit Hohlräumen nützlich sein, deren Hohlräume ansonsten nur schwer zu erreichen wären.

Zur medizinischen Behandlung von Infektionen in schwer erreichbaren Hohlräumen und Kavitäten, wie Knochenhöhlen, sind resorbierbare und nichtresorbierbare Wirkstoffträger bekannt.

Exemplarisch für nichtresorbierbare Wirkstoffträger sind die seit 1977 unter dem Markennamen Septopal^{®} bekannten Kugelketten. Diese bestehen aus Polymethylmethacrylat-Kugeln, die das Breitbandantibiotikum Gentamicinsulfat enthalten, wobei diese Kugeln auf Stahlzwirn kettenförmig angeordnet sind (K. Klemm: Gentamicin-PMMA-beads in treating bone and soft tissue infections. Zentralbl. Chir. 104(14) (1979) 934-942.; K. Klemm: Antibiotic bead chains. Clin. Orthop. 295 (1993) 63-76.). Dieser kettenförmige Wirkstoffträger (Septopal^{®}) hat sich seit Jahrzehnten bei der lokalen antibiotischen Behandlung der Osteomyelitis bewährt. Vorteilhaft ist dabei, dass das Gentamicinsulfat in größeren Mengen über einen Zeitraum von mehreren Tagen aus dem Wirkstoffträger freigesetzt wird. Vorteilhaft ist es weiterhin, dass der kettenförmige Wirkstoffträger vom medizinischen Anwender problemlos durch einfaches Abschneiden des Stahlzwirns mit überzähligen Kugeln an die anatomische Situation am Implantationsort angepasst werden kann. Nachteilig ist, dass der Wirkstoffträger ausschließlich Gentamicinsulfat enthält und dass der medizinische Anwender den Wirkstoffträger nicht mit weiteren Antibiotika, entsprechend der Empfindlichkeit der mikrobiellen Keime, modifizieren kann. Zudem kann die Abgabe des pharmazeutischen Wirkstoffs nicht mehr ohne Austausch der Kugelkette an den Verlauf der Behandlung angepasst werden, wenn die Kugelkette erst einmal implantiert ist. Dadurch ist insbesondere die erfolgreiche lokale Behandlung von Infektionen mit Problemkeimen, wie MRSA und VRSA, nur bedingt oder nicht möglich. Die Entfernung der Kugelketten nach erfolgter Wirkstofffreisetzung ist durch Verwachsung mit Bindegewebe für den Patienten mit einer erheblichen Belastung verbunden.

Exemplarisch für resorbierbare beziehungsweise biodegradierbare Wirkstoffträger sind Vliese und Schwämme aus Kollagen oder Gelatine. Exemplarisch seien dafür die Druckschriften DE 34 29 038 A1, DE 33 34 595 A1, DE 28 43 963 C2, DE 32 03 957 C2 und DE 33 34 595 A1 genannt. Diese enthalten Gentamicinsulfat oder Gemische aus Gentamicinsulfat und einem in Wasser gering löslichen Gentamicinsalz. Weiterhin gibt es eine Vielzahl von resorbierbaren beziehungsweise biodegradierbaren Wirkstoffträgern auf Basis von Tricalciumphosphat, Hydroxylapatit, Gips und deren Mischungen sowie auch Kompositmaterialien aus diesen Salzen und organischen Bindemitteln. Eine Übersicht wurde von Kühn et al. publiziert (K.-D. Kühn, N. Renz, A. Trampuz: Lokale Antibiotika-Therapie. Der Unfallchirurg. 120 (2017) 561-572).

Nachteilig an den aufgeführten nicht-resorbierbaren und auch den resorbierbaren beziehungsweise biodegradierbaren Wirkstoffträgern ist, dass der antimikrobielle Wirkstoff durch die gewählte Zusammensetzung festgelegt ist und dass nach der Implantation des Wirkstoffträgers der Wirkstoff nicht mehr ausgetauscht oder mit anderen Wirkstoffen ergänzt werden kann. Weiterhin unterliegt die Wirkstofffreisetzung bei allen bisherigen lokalen Wirkstofffreisetzungssystemen auf dem Prinzip der Diffusion, so dass hohe Wirkstoffmengen nur in den ersten Stunden oder maximal ersten Tagen freigesetzt werden. Eine Ausnahme bildet die Verwendung von in Wasser gering löslichen Wirkstoffsalzen, bei denen die Wirkstofffreisetzung vom Löslichkeitsgleichgewicht der Wirkstoffsalze abhängt.

Wünschenswert ist daher eine Vorrichtung, die eine lokale Applikation beliebiger pharmazeutischer Wirkstoffe in Form pharmazeutischer Fluide zulässt und wobei es jederzeit möglich ist, die Zusammensetzung und/oder die Konzentration an Wirkstoffen in dem pharmazeutischen Fluid auszutauschen. Außerdem ist es wünschenswert, dass die Wirkstoffkonzentration im pharmazeutischen Fluid, die unmittelbar am Implantationsort erreicht wird, direkt von außen eingestellt werden kann.

Die EP 1 932 560 B1 offenbart einen Katheter zum Applizieren eines pharmazeutischen Fluids. Der Katheter weist einen Schlauch auf, der an seinem distalen Schlauchende eine Vielzahl von Öffnungen aufweist, durch die das Fluid aus dem Inneren des Schlauchs appliziert werden kann. Weitere ähnliche Katheter sind aus der US 5 800 407 A1, der US 6 537 194 A1 und der US 5 425 723 A1 bekannt. Diese Katheter haben den Nachteil, dass eine Applikation des pharmazeutischen Fluids lediglich großflächig und in relativ hohen Mengen möglich ist. Eine lokal begrenzte Freisetzung geringer Volumina pharmazeutischer Fluide im Bereich von wenigen Mikrolitern bis zu wenigen Millilitern, beispielsweise bis zu zwei Millilitern, ist nicht möglich. Weiterhin kommt es bei den bekannten Kathetern durch Blutgerinnung und durch Einwachsen von Bindegewebe innerhalb kurzer Zeit, beispielsweise innerhalb weniger Stunden bis Tagen, zu einem Verschluss der Öffnungen, was eine Applikation eines Fluids unterbindet.

Die WO 97/37714 A1 offenbart eine selbstaufblasende Katheter- und Ballonanordnung mit einer autoregulierenden Struktur, um ein Überblasen des Ballons als Ergebnis variabler Flüssigkeitsflussraten durch das Katheterlumen zu verhindern. Am distalen Ende des Katheterkörpers ist ein eng anliegender Elastomerballon vorgesehen, und die Anordnung ist so konstruiert, dass zumindest ein Teil des Flüssigkeitsstroms durch das Lumen zum Ballon geleitet wird, um ihn aufzublasen. Wenn der Ballon aufgeblasen wird, wird mehr und mehr des Flüssigkeitsstroms durch den Katheter aus dem Katheter abgeleitet, wodurch ein Überblähen des Ballons verhindert wird.

Die FR 2 707 505 A1 offenbart einen Katheter mit einem Katheterschlauch, der einen inneren, sich entlang einer Achse erstreckenden Kanal enthält, und mindestens einem diesem Schlauch zugeordneten Zweiwegeventil zur selektiven Verbindung zwischen seinem Kanal und der Außenwelt. Das Ventil, das im Ruhezustand normalerweise geschlossen ist, im Wesentlichen entlang der Achse der Leitung gegenüberliegend angeordnet.

Die DE 92 09 164 U1 offenbart einen Katheter zur Injektion von Flüssigkeit in den menschlichen oder tierischen Körper, bei dem an dem zur Einführung bestimmten Ende ein Mikroventil für die Steuerung der Flüssigkeitsausströmung angeordnet ist.

Die DE 10 2013 110989 A1 offenbart einen Ballonkatheter, umfassend eine Katheterleitung, einen dehnbaren Ballon, der einen Balloninnenraum berandet und der an der Katheterleitung ausgebildet ist, und eine Anschlusseinrichtung, die zumindest einen ersten Anschluss zur Zufuhr von Fluid, bspw. von Luft, aufweist und die an der Katheterleitung angeordnet ist.

Die EP 0 191 154 A2 offenbart einen Katheter für die Dauerapplikation von Pharmaka mit einem an der Katheterspitze angeordneten Einwegeventil, welches als Entenschnabelventil ausgebildet ist. Dasselbe weist vorzugsweise zur Befestigung am Katheterschlauch einen Stutzen auf, der auf oder in den Katheterschlauch geschoben wird und entweder aufgrund seiner Elastizität oder durch Verkleben oder Verschweißen haftet.

### Aufgaben

Eine Aufgabe der vorliegenden Erfindung ist es, einen oder mehrere der sich aus dem Stand der Technik ergebenen Nachteile zumindest teilweise zu überwinden.

Insbesondere soll eine Vorrichtung zur lokalen Applikation kleiner Mengen pharmazeutischer Fluide, wie zum Beispiel von antibiotischen Lösungen, bereitgestellt werden, mit der eine lokale und temporäre Abgabe des pharmazeutischen Fluids in kleinvolumigen, schwer zugänglichen Bereichen ermöglicht wird. Die Vorrichtung soll dabei flexibel an unterschiedliche Anwendungsbereiche anzupassen sein. Eine mechanische Belastung der Wandungen der zu behandelnden Hohlräume soll dabei möglichst vermieden werden. Bei der Verwendung zur Behandlung einer Infektion soll eine möglichst schonende Behandlung möglich sein, bei der das angrenzende entzündete Gewebe möglichst wenig gereizt wird und zwar sowohl bei einer temporären Abgabe des Fluids als auch beim Einsetzen und Entfernen des eingeführten Teils der Vorrichtung. Die Vorrichtung soll auch über längere Zeiträume an einem bestimmten Ort zur wiederholten Abgabe des Fluids geeignet sein, ohne dass die Vorrichtung hierfür entfernt werden muss. Die Vorrichtung soll kostengünstig zu fertigen sein und möglichst ein hygienisches, nur einmal verwendbares Wegwerfprodukt sein. Dabei soll zumindest der in den zu behandelnden Hohlraum platzierbare Teil der Vorrichtung oder eben die gesamte Vorrichtung als Wegwerfprodukt kostengünstig und leicht entsorgbar sein. Gleichzeitig soll das pharmazeutische Fluid aber nicht unkontrolliert aus der Vorrichtung ausfließen können. Die Vorrichtung soll durch ein selbsttätiges Verschließen der Austragsöffnung nach erfolgter Freisetzung der pharmazeutischen Fluide gegen einen Verschluss der Austragsöffnung, beispielsweise durch Einwachsen von Bindegewebe oder durch geronnenes Blut, geschützt sein. Die Flüssigkeitsabgabe aus der Vorrichtung soll von außen kontrolliert werden können.

Die Aufgabe der Erfindung besteht somit auch darin, eine einfache, kostengünstige Vorrichtung zur lokalen Applikation von pharmazeutischen Fluiden zu entwickeln. Die Vorrichtung soll eine lokale Applikation insbesondere von pharmazeutischen Fluiden mit beliebiger Zusammensetzung, zum Beispiel antibiotischen Lösungen, ermöglichen. Ein Teil der Vorrichtung befindet sich bei einer medizinischen Anwendung nach einer Implantation im Patienten und ein zweiter Teil der Vorrichtung außerhalb des Patienten. Die pharmazeutischen Fluide sollen in dem außerhalb des im Patienten befindlichen Teils der Vorrichtung eingebracht werden können und zum Implantationsort durch die Vorrichtung geleitet und dort freigesetzt werden können. Die Vorrichtung soll plastisch verformbar sein, um den anatomischen Gegebenheiten am Implantationsort oder der geometrischen Form der Hohlform folgen zu können.

Es ist eine weitere Aufgabe der Erfindung, ein System bereitzustellen, mit dem eine lokal begrenzte Applikation von kleinen Mengen pharmazeutischer Fluide möglich ist, mittels dem mindestens ein Teil der bereits beschriebenen Aufgaben zumindest zum Teil gelöst wird.

Es ist eine weitere Aufgabe der Erfindung, ein Verfahren bereitzustellen, mit dem eine lokal begrenzte Applikation von kleinen Mengen pharmazeutischer Fluide möglich ist, mittels dem mindestens ein Teil der bereits beschriebenen Aufgaben zumindest zum Teil gelöst wird.

### Bevorzugte Ausführungsformen der Erfindung

Ein Beitrag zur mindestens teilweisen Erfüllung mindestens einer der zuvor genannten Aufgaben wird durch die Merkmale der unabhängigen Ansprüche geleistet. Die abhängigen Ansprüche stellen bevorzugte Ausführungsformen bereit, die zur mindestens teilweisen Erfüllung mindestens einer der Aufgaben beitragen.

### Allgemeines

In der vorliegenden Beschreibung beinhalten Bereichsangaben auch die als Grenzen genannten Werte. Eine Angabe der Art "im Bereich von X bis Y" in Bezug auf eine Größe A bedeutet folglich, dass A die Werte X, Y und Werte zwischen X und Y annehmen kann. Einseitig begrenzte Bereiche der Art "bis zu Y" für eine Größe A bedeuten entsprechend als Wert Y und kleiner als Y.

Einige der beschriebenen Merkmale sind mit dem Begriff "im Wesentlichen" verknüpft. Der Begriff "im Wesentlichen" ist so zu verstehen, dass unter realen Bedingungen und Fertigungstechniken eine mathematisch exakte Auslegung von Begrifflichkeiten wie "Überlagerung", "senkrecht", "Durchmesser" oder "Parallelität" nie exakt, sondern nur innerhalb gewisser fertigungstechnischer Fehlertoleranzen gegeben sein kann. Beispielsweise schließen "im Wesentlichen senkrechte Achsen" einen Winkel von 85 Grad bis 95 Grad zueinander ein und "im Wesentlichen gleiche Volumen" umfassen eine Abweichung von bis zu 5 Volumen-%. Eine "im Wesentlichen aus Kunststoff bestehende Vorrichtung" umfasst beispielsweise einen Kunststoffanteil von ≥95 bis ≤100 Gewichts-%. Eine "im Wesentlichen vollständige Befüllung eines Volumens B" umfasst beispielsweise eine Befüllung von ≥95 bis ≤100 Volumen-% des Gesamtvolumens von B.

### Ausführliche Beschreibung

Ein erster Gegenstand der Erfindung betrifft eine Vorrichtung zur Applikation eines pharmazeutischen Fluids umfassend einen Schlauch und ein Ventilelement,
wobei der Schlauch über ein erstes Schlauchende mit einem Reservoir für das pharmazeutische Fluid fluidleitend verbindbar ist und wobei das Ventilelement in einem zweiten Schlauchende des Schlauchs angeordnet ist,
wobei das Ventilelement an einem dem ersten Schlauchende zugewandten ersten Ventilelementende mindestens ein Verankerungselement aufweist, um ein Ausbringen des Ventilelements aus dem zweiten Schlauchende zu verhindern,
an einem dem zweiten Schlauchende zugewandten zweiten Ventilelementende ein Dichtungselement mit einem Dichtungselementaußendurchmesser aufweist, welcher zumindest einem Schlauchinnendurchmesser des Schlauchs entspricht, so dass das zweite Schlauchende durch das Dichtungselement fluidleitend verschlossen ist, und
ein Leitungsmittel umfasst, welches das erste Schlauchende und das Dichtungselement fluidleitend verbindet, wobei bei einer Druckbeaufschlagung auf das pharmazeutische Fluid aus Richtung des ersten Schlauchendes reversibel ein Spalt zwischen dem Schlauch und dem Dichtungselement ausformbar ist, so dass das zweite Schlauchende fluidleitend geöffnet wird.

Das Ventilelement verschließt das zweite Schlauchende fluidleitend, bis ein derart großer Druck aus Richtung des ersten Schlauchendes auf ein pharmazeutisches Fluid ausgeübt wird, dass sich der Schlauch im Bereich des zweiten Schlauchendes, insbesondere auf Höhe des Dichtungselements, durch eine Einwirkung des pharmazeutischen Fluids radial derart ausdehnt, dass ein Spalt zwischen Schlauch und Dichtungselement entsteht, durch welchen das pharmazeutische Fluid aus der Vorrichtung ausbringbar ist. Ist die Druckbeaufschlagung niedriger, ist diese insbesondere im Bereich des Normaldrucks, wirken das Dichtungselement und eine Wandung, insbesondere eine Innenwandung, des Schlauchs derart zusammen, dass das zweite Schlauchende fluidleitend verschlossen ist und das pharmazeutische Fluid nicht aus der Vorrichtung ausbringbar ist. Der Spalt bildet sich somit bei einem Innendruck aus, der ausreichend groß ist, um den Schlauch zumindest auf Höhe des Dichtungselements radial zu dehnen. Durch den unter Druckbeaufschlagung entstandenen Spalt ist das zweite Schlauchende fluidleitend geöffnet, so dass das pharmazeutische Fluid aus der Vorrichtung gezielt aus der Vorrichtung appliziert werden kann. Der Spalt kann sich dabei ringförmig oder auch nur teilweise um das Dichtungselement ausbilden. Je größer die Druckbeaufschlagung, desto eher bildet sich der Spalt ringförmig um das Dichtungselement aus. Mit nachlassendem Druck, insbesondere verursacht durch ein Applizieren des Fluids aus der Vorrichtung heraus, zieht sich der Schlauch wieder radial zusammenziehen, wodurch der Spalt geschlossen wird. Das Schließen des Spalts erfolgt dabei innerhalb weniger Augenblicke, beispielsweise innerhalb von Bruchteilen einer Sekunde. Dadurch ist ein Einwachsen von Bindegewebe und ein Eindringen von Blut in die Vorrichtung unterbunden, weshalb die Vorrichtung funktionsfähig über mehrere Tage bis Wochen im Patientenkörper implantiert sein kann und dabei eine wiederholte lokale Applikation eines pharmazeutischen Fluids an der gewünschten Stelle im Patienten ermöglicht ist. Ein weiterer Vorteil des Aufbaus der Vorrichtung ist, dass, selbst falls das zweite Schlauchende, beispielsweise durch Bindegewebe oder geronnenes Blut, fluidleitend verstopft sein sollte, diese Verstopfung durch die Druckbeaufschlagung und die darauffolgende Ausbringung des pharmazeutischen Fluids aus der Vorrichtung unter Druck beseitigt würde. Der Aufbau und die Funktionsweise der Vorrichtung ermöglicht somit eine "Selbstreinigung" der Verstopfung durch Gebrauch der Vorrichtung.

Dabei kann die Vorrichtung auf zumindest zwei Arten betrieben werden. In einer ersten Art wird die Druckbeaufschlagung pulsartig ausgeführt, so dass bereits durch ein Applizieren einer kleinen Menge an pharmazeutischem Fluid, beispielsweise bis zu 2 Milliliter, aus der Vorrichtung heraus der Innendruck derart abnimmt, dass der Spalt wieder geschlossen wird. Dabei erreicht die Druckbeaufschlagung nur kurzfristig, also pulsartig, die benötigte Höhe, um den Spalt reversibel auszuformen. In einer weiteren Art erfolgt eine kontinuierliche Druckbeaufschlagung auf das pharmazeutische Fluid, so dass dieses so lange aus dem zweiten Schlauchende austragbar ist, solange die Druckbeaufschlagung mit der benötigten Höhe aufrechterhalten wird. Diese Art der Druckbeaufschlagung erlaubt somit eine kontinuierliche Applikation des pharmazeutischen Fluids.

Die Vorrichtung umfasst einen Schlauch. Unter einem Schlauch ist ein flexibel verformbarer Hohlkörper mit einer einen Innenraum umgebenden Wandung zu verstehen, durch den ein Fluid, insbesondere ein pharmazeutisches Fluid, von einem fluidleitend offenen ersten Schlauchende zu einem fluidleitend offenen zweiten Schlauchende förderbar ist. Der Innenraum kann dabei unterschiedlich ausgestaltete Querschnittsgeometrien, wie beispielsweise oval oder auch mehreckig aufweisen. Aufgrund der einfachen Herstellung und Verwendung ist ein im Wesentlichen runder Querschnitte des Innenraums bevorzugt. Um ein kontrolliertes Applizieren auch kleiner Mengen an pharmazeutischem Fluid, beispielsweise bis zu 2 Millilitern, zu ermöglichen, ist es bevorzugt, dass der Schlauch einen Schlauchinnendurchmesser, also der Durchmesser des Innenraums, in einem Bereich von 0,5 bis 3 mm, bevorzugt in einem Bereich von 0,8 bis 2,5 mm, weiter bevorzugt von 1 bis 2 mm aufweist.

Die Vorrichtung umfasst ein Ventilelement. Das Ventilelement ist zumindest teileweise innerhalb des zweiten Schlauchendes angeordnet, so dass dieses durch das Ventilelement solange fluidleitend verschlossen ist, bis ein ausreichend hoher Druck auf das pharmazeutische Fluid ausgeübt wird, dass reversibel der Spalt entsteht und das pharmazeutische Fluid aus der Vorrichtung appliziert werden kann.

Um das zweite Schlauchende fluidleitend zu verschließen, umfasst das Ventilelement ein zumindest teilweise innerhalb des zweiten Schlauchendes angeordnetes Dichtungselement. Das Dichtungselement füllt den Innenraum des Schlauchs am zweiten Schlauchendes derart aus, dass ohne Druckbeaufschlagung die Wandung des Schlauchs fluidleitend verschlossen an einer Außenfläche des Dichtungselements anliegt. Dazu weist das Dichtungselement einen Dichtungselementaußendurchmesser auf, welcher zumindest dem Schlauchinnendurchmesser des Schlauchs entspricht. Bevorzugt weist der Schlauch einen runden Innenraumquerschnitt auf, weshalb das Dichtungselement ebenso bevorzugt einen runden Querschnitt aufweist.

Weisen Schlauchinnendurchmesser und Dichtungselementaußendurchmesser den gleichen Betrag auf, liegt der Schlauch in einem entspannten, ungedehnten Zustand am Dichtungselement an, so dass das zweite Schlauchende fluidleitend verschlossen sind. Ist der Dichtungselementaußendurchmesser größer als der Schlauchinnendurchmesser, dehnt das Dichtungselement den Schlauch, insbesondere das zweite Schlauchende, so dass der Schlauch in einem gedehnten Zustand am Dichtungselement anliegt um das zweite Schlauchende fluidleitend zu verschließen. Um Produktionsungenauigkeiten und Materialermüdung vorzubeugen, ist es bevorzugt, dass der Dichtungselementaußendurchmesser größer ist als der Schlauchinnendurchmesser. Bevorzugt ist der Dichtungselementaußendurchmesser mindestens 5%, weiter bevorzugt mindestens 10 %, am bevorzugtesten mindestens 15 % größer als der Schlauchinnendurchmesser. Um ein Einreißen des Schlauchs und Materialermüdung vorzubeugen, ist es bevorzugt, dass der Dichtungselementdurchmesser nicht mehr als 30% größer ist als der Schlauchinnendurchmesser. Zudem erhöht sich die benötigte Druckbeaufschlagung auf das pharmazeutische Fluid zur Ausbildung des Spalts mit zunehmendem Größenunterschied von Dichtungselementaußendurchmesser zu Schlauchinnendurchmesser durch die damit einhergehende Dehnung des zweiten Schlauchendes im Ausgangzustand bereits ohne ausgebildeten Spalt, weshalb der Dichtungselementdurchmesser bevorzugt nicht mehr als 30% größer ist als der Schlauchinnendurchmesser. Dabei sind die Wertangaben so zu verstehen, dass beispielsweise die Angabe "der Ventilelementaußendurchmesser ist 30% größer als der Schlauchinnendurchmesser" bedeutet, dass der Ventilelementaußendurchmesser 130% des Schlauchinnendurchmessers entspricht.

Damit das Ventilelement derart fest im zweiten Schlauchende befestigt ist, dass ein, insbesondere unbeabsichtigtes, Ausbringen des Ventilelements aus dem zweiten Schlauchende, beispielsweise im Zuge der Druckbeaufschlagung auf das pharmazeutische Fluid zur Ausbildung des Spalts, verhindert wird, weist das Ventilelement an einem ersten Ventilelementende ein Verankerungselement auf. Das Verankerungselement wirkt mit der Wandung des Schlauchs derart zusammen, dass das Ventilelement nach dem Einbringen in das zweite Schlauchende nicht zerstörungsfrei aus dem zweiten Schlauchende herausgezogen beziehungsweise herausgedrückt werden kann. Unter dem ersten Ventilelementende ist nicht nur die äußere "Spitze" des Ventilelements zu verstehen, welche in Richtung des ersten Schlauchendes deutet. Vielmehr erstreckt sich das erste Ventilelementende aus Richtung des ersten Schlauchendes gesehen über 2/3 der gesamten axialen Erstreckung des Ventilelements.

Damit das pharmazeutische Fluid aus Richtung des ersten Schlauchendes bis zum Dichtungselement am zweiten Ventilelementende gelangen kann, um dort unter Druckbeaufschlagung den Spalt auszubilden, weist das Ventilelement ein Leitungsmittel auf. Das Leitungsmittel ist ein fluidleitendes Element, durch welches das pharmazeutische Fluid aus Richtung des ersten Schlauchendes am Verankerungselement vorbei, bis zum Dichtungselement am zweiten Ventilelementende förderbar ist. Das Leitungsmittel sorgt dafür, dass durch die Druckbeaufschlagung auf das pharmazeutische Fluid der Spalt im Bereich des Dichtungselements ausgebildet wird und es nicht zu einer Dehnung des Schlauchs im Bereich des mindestens einen Verankerungselements kommt.

Die erfindungsgemäße Vorrichtung dient zur lokalen Applikation eines pharmazeutischen Fluids, insbesondere zur Behandlung infizierten Gewebes, wie beispielsweise Weich- und/oder Knochengewebes, über einen Zeitraum von mehreren Tagen bis Wochen. Ein pharmazeutisches Fluid enthält wenigstens einen pharmazeutischen Wirkstoff. Beispielsweise handelt es sich bei dem pharmazeutischen Fluid um wässrige oder nicht-wässrige Lösungen oder Suspensionen von pharmazeutischen Wirkstoffen.

In einer Ausgestaltungsform handelts es sich bei dem pharmazeutisches Fluid um Lösungen, welche wenigstens ein Antibiotikum, wenigstens ein Chemotherapeutikum und/oder wenigstens ein Antimykotikum enthalten. In einer weiteren Ausgestaltungsform enthalten die pharmazeutischen Fluide wenigstens einen desinfizierenden Bestandteil.

Weiterhin umfassen pharmazeutische Fluide auch Gase, Gasgemische und Lösungen von Gasen in Flüssigkeiten, wie beispielsweise Wasser.

Die zur Ausbildung des Spalts zwischen Dichtungselement und Schlauch benötigte Druckbeaufschlagung auf das pharmazeutische kann von unterschiedlichen Faktoren, wie beispielsweise dem Material des Schlauchs, dem Schlauchinnendurchmesser, dem Verhältnis von Dichtungselementdurchmesser zu Schlauchinnendurchmesser und der Länge des Dichtungselements, abhängen.

Um ein kontrolliertes Applizieren des pharmazeutischen Fluids aus der Vorrichtung zu gewähren, ist eine Ausführungsform der Vorrichtung dadurch gekennzeichnet, dass erst ab einer Druckbeaufschlagung von mindestens 5 N/cm² auf das pharmazeutische Fluid aus Richtung des ersten Schlauchendes reversibel ein Spalt zwischen dem Schlauch und dem Dichtungselement ausformbar ist, so dass das zweite Schlauchende fluidleitend geöffnet ist. Erreicht die Druckbeaufschlagung nicht diesen Grenzwert, bleibt das zweite Schlauchende fluidleitend geschlossen. Dies verhindert ein unbeabsichtigtes Austreten des pharmazeutischen Fluids aus dem Schlauch in den Patienten, welches gesundheitliche Risiken nach sich ziehen könnte. Damit ein Applizieren kontrolliert, in kleinen Mengen und ohne das Risiko einer Rissbildung im Schlauch stattfinden kann, ist es bevorzugt, dass zur Spaltbildung die Druckbeaufschlagung nicht mehr als 50 N/cm² betragen muss.

Das Ventilelement weist eine Ventilelementlänge auf. Um ein funktionstüchtiges Applizieren mit der Vorrichtung zu gewährleisten, ist die Ventilelementlänge an den Dichtungselementaußendurchmesser anzupassen. Je größer der Dichtungselementaußendurchmesser desto größer ist die Ventilelementlänge zu wählen.

Eine Ausführungsform der Vorrichtung ist dadurch gekennzeichnet, dass das Ventilelement eine Ventilelementlänge aufweist, welche mindestens dreimal dem Dichtungselementaußendurchmesser entspricht. Ist die Vorrichtung kürzer, kann es bei einer Druckbeaufschlagung oder bereits bei der Montage der Vorrichtung zu einem "Verkippen" des Ventilelements innerhalb des Schlauchs kommen. Dadurch wäre die Vorrichtung am zweiten Schlauchende nicht fluidleitend verschlossen. Um die Vorrichtung möglichst flexibel auszugestalten, ist es bevorzugt, dass die Ventilelementlänge nicht mehr als dem fünffachen des Dichtungselementaußendurchmessers entspricht.

Um das Ventilelement sicher im zweiten Schlauchende zu befestigen, so dass dieses im Zuge der Druckbeaufschlagung nicht aus dem zweiten Schlauchende ausgetragen wird, können unterschiedliche Verankerungselemente zum Einsatz kommen. In einer ersten Ausgestaltungsform kann das Verankerungselement ein Befestigungsmittel, wie beispielsweise eine Schraube sein, welches das Ventilelement und den Schlauch miteinander verbindet. In einer weiteren Ausgestaltungsform kann das Verankerungselement als Nut-Feder-Verbindung zwischen Ventilelement und Schlauch ausgestaltet sein, wobei ein Element die Nut und das andere Element die Feder aufweist.

Eine Ausführungsform der Vorrichtung ist dadurch gekennzeichnet, dass das mindestens eine Verankerungselement mindestens ein Widerhakenelement ist. Ein Widerhakenelement erlaubt ein einfaches Einbringen des Ventilelements in das zweite Schlauchende und verhindert gleichzeitig effektiv ein Austragen aus der Vorrichtung in die entgegengesetzte Richtung. Gleichzeitig ist ein Widerhakenelement produktionstechnisch einfach und günstig umsetzbar. Das mindestens eine Widerhakenelement weist dabei ein der Wandung des Schlauchs zugewandtes spitzes Ende auf, welches durch Zusammenwirken mit der Wandung verhindert, dass das eingeführte Ventilelement aus dem zweiten Schlauchende herausgedrückt wird.

Das Widerhakenelement kann unterschiedlich ausgeformt sein. In einer Ausführungsform weist das Ventilelement mindestens einen hakenförmig gebogenen Draht, ähnlich einem Angelhacken, auf, welcher das Ventilelement innerhalb des Schlauchs befestigt. In einer weiteren Ausführungsform weist das Ventilelement mindestens einen schräg in Richtung des zweiten Ventilelementendes abstehenden Dorn oder Stab auf, welcher als Widerhakenelement fungiert.

Eine Ausführungsform der Vorrichtung ist dadurch gekennzeichnet, dass das mindestens eine Widerhakenelement als Konus mit einer Konusbasis und einem Konusmantel ausgeformt ist, wobei der Konusmantel dem ersten Ventilelementende und die Konusbasis dem zweiten Ventilelementende zugewandt ist. Der Konus ist bevorzugt rotationssymmetrisch ausgeformt, wobei die Längsachse des Konus und die Längsachse des Ventilelements bevorzugt deckungsgleich liegen. In einem Längsschnitt durch das Ventilelement ist der Konus als dreiecksförmige Erhebung auf einem Grundkörper des Ventilelements ausgebildet. In einer Ausgestaltungsform verläuft der Konus vollständig um das Ventilelement herum. In einer weiteren Ausgestaltungsform ist der Konus an einer oder mehreren Stellen durchbrochen.

Eine Ausführungsform der Vorrichtung ist dadurch gekennzeichnet, dass die Konusbasis und der Konusmantel einen Winkel von maximal 80°, bevorzugt von maximal 75°, weiter bevorzugt von maximal 70°, am bevorzugtesten von maximal 65 ° einschließen. Dies erlaubt eine sichere Befestigung des Ventilelements innerhalb des zweiten Schlauchendes bei gleichzeitig einfachen Einbringen des Ventilelements in den Schlauch.

Um das Risiko eines Ausbringens des Ventilelements weiter zu senken, ist es bevorzugt, dass mindestens zwei Verankerungselemente, bevorzugt Widerhakenelemente, hintereinander in axialer Ausrichtung des Ventilelements angeordnet sind.

Bevorzugt ist das Ventilelement einstückig ausgebildet, wobei das Ventilelement ein biokompatibles Metall, eine biokompatible Metalllegierung und/oder einen biokompatiblen Kunststoff umfasst oder aus einem der vorgenannten Materialien oder Materialkombinationen besteht. Beispiele für Kunststoffe umfassen Polyamide, Polyester, Polyketone, Polymethacrylate und deren Copolymere. Beispiels für Metalle und Metalllegierungen umfassen Edelstähle, wie Edelstahl 1.4404, oder Titanlegierungen, wie TiAl₆V₄.

In einer Ausführungsform verläuft das Leitungsmittel rohrartig vom ersten Ventilelementende in Richtung des zweiten Ventilelementendes, wo es räumlich vor dem Dichtungselement heraustritt, so dass das pharmazeutische Fluid vom ersten Schlauchende bis zum Dichtungselement, insbesondere zur Kontaktstelle von Dichtungselement und Wandung des Schlauchs förderbar ist, um bei Druckbeaufschlagung den Spalt auszubilden.

Eine Ausführungsform der Vorrichtung ist dadurch gekennzeichnet, dass der Konus zumindest an einer Stelle durch das Leitungsmittel durchbrochen ist, so dass das das erste Schlauchende über das Leitungsmittel durch den Konus mit dem Dichtungselement fluidleitend verbunden ist. Das Leitungsmittel ist bei dieser Ausführungsform auf einer Außenfläche des Ventilelements ausgebildet, was die produktionstechnische Fertigung des Ventilelements erleichtert.

Der Schlauch kann ein oder mehrere unterschiedliche Materialien umfassen oder daraus bestehen, sofern die Ausformung des Spalts kontrolliert, sicher und reversibel zur Applikation eines pharmazeutischen Fluids möglich ist.

Eine Ausführungsform der Vorrichtung ist dadurch gekennzeichnet, dass der Schlauch ein elastisches Polymer mit einer Shore A-Härte von größer 50 oder ein thermoplastisches Polymer mit einer Shore D-Härte von größer 10 umfasst, insbesondere das der Schlauch aus einem elastischen Polymer mit einer Shore A-Härte von größer 50 oder einem thermoplastischen Polymer mit einer Shore D-Härte von größer 10 besteht. Dies ermöglicht eine sichere, kontrollierte und wiederholbare Applikation eines pharmazeutischen Fluids, insbesondere auch in kleinen Mengen, wie beispielsweise bis zu 2 Millimetern. Die zur Ausbildung des Spalts benötigte Druckbeaufschlagung kann bei gegebenen Schlauchinnendurchmesser und Dichtungselementaußendurchmesser durch die Wahl des Schlauchmaterials eingestellt werden.

Um eine sachgerechte und zielgerichtete Applikation an der gewünschten Stelle innerhalb eines Patienten zu kontrollieren, ist eine Ausführungsform der Vorrichtung dadurch gekennzeichnet, dass der Schlauch einen Röntgenopaker aufweist. Durch einen Röntgenopaker kann die richtige Positionierung der Vorrichtung innerhalb des Patienten über bildgebende Verfahren mittels Röntgenstrahlung (X-ray) sichtbar gemacht werden. Beispiele für Röntgenopaker sind Bariumsulfat, Zirkondioxid und Kalziumcarbonat.

Durch die flexible Verformbarkeit des Schlauchs ist es mittels der Vorrichtung möglich, ein pharmazeutisches Fluid auch an schwer zugänglichen Stellen innerhalb des Patienten zu applizieren. Zudem erlaubt die Flexibilität des Schlauchs eine weitgehend schmerzfreie Beweglichkeit des Patienten trotz implantierter Vorrichtung.

Um der Vorrichtung, insbesondere dem Schlauch, trotz der flexiblen Verformbarkeit des Schlauchs eine gewisse Stabilität zu geben, beispielsweise um eine Implantation in den Patienten zu erleichtern, kann es bevorzugt sein, den Schlauch strukturell zu verstärken. In einer Ausführungsform geschieht dies durch den Einsatz eines oder mehrerer Drähte, insbesondere eines oder mehrere Metalldrähte, welche in der Wandung des Schlauchs und/oder im Innenraum des Schlauchs parallel zur Längsachse des Schlauchs angeordnet sind.

Eine Ausführungsform der Vorrichtung ist dadurch gekennzeichnet, dass innerhalb des Schlauchs und/oder in der Wandung des Schlauchs mindestens eine Metallspirale entlang einer Längsachse der Vorrichtung angeordnet ist. Dabei haben Metallspirale und Schlauch deckungsgleiche Längsachsen. Der Innenraum des Schlauchs ist von der Metallspirale "umwickelt". Ein Vorteil einer Metallspirale ist, dass die Flexibilität der Vorrichtung erhalten bleibt und gleichzeitig die Vorrichtung in eine gewünschte Form gebogen werden kann, beispielsweise um die Implantation in den Patienten zu erleichtern.

Die Vorrichtung, insbesondere das erste Schlauchende, kann auf unterschiedliche Weisen mit einem Reservoir für ein pharmazeutisches Fluid fluidleitend verbindbar oder verbunden sein. In einer ersten Ausgestaltungsform kann das erste Schlauchende auf eine Tülle eines Reservoirs aufgesteckt werden. In einer weiteren Ausführungsform sind Schlauch und Reservoir mittels einer Gewindeverbindung fluidleitend verbindbar oder verbunden. In weiteren Ausführungsformen sind Schlauch und Reservoir mittels einer Flanschverbindung oder über eine Schlauchverbindung fluidleitend miteinander verbindbar oder verbunden.

Eine Ausführungsform der Vorrichtung ist dadurch gekennzeichnet, dass die Vorrichtung einen Adapter aufweist, mit dem die Vorrichtung und ein Reservoir für ein pharmazeutisches Fluid fluidleitend, insbesondere reversibel fluidleitend, verbindbar sind. Ein Vorteil eines Adapters ist, dass die Vorrichtung mit einer Vielzahl an unterschiedlich Reservoiren fluidleitend verbindbar ist, unabhängig von der jeweiligen Ausgestaltung und Durchmessers des vorhandenen Fluidauslasses am Reservoir.

Eine Ausführungsform der Vorrichtung ist dadurch gekennzeichnet, dass die Vorrichtung innerhalb des Schlauchs, insbesondere angeordnet zwischen erstem Schlauchende und Dichtungselement, ein Rückschlagventil aufweist, welches in Richtung des ersten Schlauchendes fluidundurchlässig ausgestaltet ist. Das Rückschlagventil kann ein Rückfließen eines pharmazeutischen Fluids in ein mit der Vorrichtung am zweiten Schlauchende fluidleitend verbundenes Reservoir unterbinden, was eine Kontamination des Reservoirs verhindert.

Das Rückschlagventil kann unterschiedliche Bauformen aufweisen. In einer Ausführungsform umfasst das Rückschlagventil eine Rückschlagklappe, welche ein Fördern des pharmazeutischen Fluids durch den Schlauch nur in einer Flussrichtung erlaubt und in die entgegengesetzte Flussrichtung den Schlauch fluidleitend verschließt. In einer weiteren Ausführungsform weist das Rückschlagventil ein Rückstellelement, beispielsweise eine Feder, insbesondere eine Spiralfeder, auf, welches das Rückschlagventil in Richtung des ersten Schlauchendes fluidleitend verschließt und in die entgegengesetzte Richtung ein Fördern des pharmazeutischen Fluids durch den Schlauch erlaubt. In einer weiteren Ausführungsform ist das Rückschlagventil als Kugelrückschlagventil ausgebildet. Aufgrund der hohen strukturellen Robustheit ist in einer weiteren, bevorzugten, Ausgestaltungsform das Rückschlagventil als Tellerrückschlagventil ausgebildet.

Ein weiterer Gegenstand der Erfindung betrifft ein System zur Applikation eines pharmazeutischen Fluids umfassend eine Vorrichtung nach einer der vorhergehenden Ausführungsformen. Neben der Vorrichtung umfasst das System ein Reservoir für das pharmazeutische Fluid, wobei der Schlauch der Vorrichtung über das erste Schlauchende mit dem Reservoir fluidleitend verbindbar oder verbunden ist.

Unter einem Reservoir werden alle Behältnisse verstanden, welche dazu geeignet sind, das pharmazeutische Fluid zu lagern. Beispiele für Reservoire umfassen Beutel, Spritzen, Kolben, Ballons, Kanister und Ampullen, wobei Beutel, Ballons und Spritzen bevorzugt sind.

Die Druckbeaufschlagung auf das pharmazeutische Fluid zur Ausbildung des Spalts kann auf unterschiedliche Arten ausgeübt werden. In einer Ausführungsform ist das System, insbesondere der Schlauch der Vorrichtung, mit einer separaten Pumpe, insbesondere einer Peristaltikpumpe, verbunden, welche die Druckbeaufschlagung auf das pharmazeutische Fluid ausüben kann.

Eine Ausführungsform des Systems ist dadurch gekennzeichnet, dass das Reservoir ein Fördermittel zum Fördern eines pharmazeutischen Fluids aus dem Reservoir in das erste Schlauchende und zum Erzeugen der Druckbeaufschlagung auf das pharmazeutische Fluid aus Richtung des ersten Schlauchendes umfasst. In einer Ausführungsform des Systems ist das Reservoir eine Spritze und das Fördermittel ein axial in der Spritze verschiebbarer Kolben.

Ein weiterer Gegenstand der Erfindung betrifft ein Verfahren zur Applikation eines pharmazeutischen Fluids mit einer Vorrichtung nach einer der vorhergehenden Ausführungsformen aufweisend die folgenden Schritte:
a. Fluidleitendes Verbinden der Vorrichtung mit dem Reservoir für das pharmazeutische Fluid,
b. Fördern des pharmazeutischen Fluids aus dem Reservoir über den Schlauch bis zum Dichtungselement,
c. Aufbau eines Drucks auf das pharmazeutische Fluid aus Richtung des ersten Schlauchendes innerhalb des Schlauchs von größer 5 N/cm²,
d. Radiale Dehnung des Schlauchs im Bereich des Dichtungselements durch Einwirkung des Drucks unter Ausbildung eines Spalts zwischen Dichtungselement und Schlauch,
e. Ausbringen des pharmazeutischen Fluids aus dem Spalt unter Abbau des Drucks,
f. Radiales Zusammenziehen des Schlauchs unter Schließen des Spalts zwischen Dichtungselement und Schlauch.

Unter einem Reservoir werden alle Behältnisse verstanden, welche dazu geeignet sind, das pharmazeutische Fluid zu lagern. Beispiele für Reservoire umfassen Beutel, Spritzen, Kolben, Ballons, Kanister und Ampullen, wobei Beutel, Ballons und Spritzen bevorzugt sind.

Das fluidleitende Verbinden von Vorrichtung und Reservoir in Schritt a. kann zu unterschiedlichen Zeitpunkten erfolgen. In einer Ausführungsform findet das Verbinden unmittelbar, beispielsweise bis zu 5 Minuten, vor der Applikation des pharmazeutischen Fluids in den Patienten, statt. In einer weiteren Ausführungsform findet das Verbinden bereits weit vor der Applikation des pharmazeutischen Fluid statt, beispielsweise bereits mehrere Tage bis mehrere Monate vor der Applikation des pharmazeutischen Fluids. Beispielsweise kann das fluidleitende Verbinden bereits bei der Herstellung der Vorrichtung erfolgen.

In Schritt b. erfolgt ein Fördern des pharmazeutischen Fluids aus dem Reservoir über den Schlauch bis zum Dichtungselement, so dass der Schlauch vom ersten Schlauchende bis zum Dichtungselement im Wesentlichen vollständig mit dem pharmazeutischen Fluid gefüllt ist. Das Fördern findet bevorzugt unter Ausübung eines Drucks auf das pharmazeutische Fluid statt.

Um das pharmazeutische Fluid aus der Vorrichtung, insbesondere aus dem zweiten Schlauchende, zu fördern und an einer gewünschten Stelle zu applizieren, wird in Schritt c. ein Druck von größer 5 N/cm² auf das pharmazeutische Fluid aus Richtung des ersten Schlauchendes in Richtung des Dichtungselements ausgeübt. Ein derartig hoher Druck sorgt in Schritt c. zur reversiblen Ausbildung eines Spalts zwischen Dichtungselement und Schlauch durch Ausdehnung des Schlauchs. Ist der Druck geringer, kommt es zu keiner radialen Dehnung des Schlauchs und das zweite Schlauchende bleibt fluidleitend verschlossen.

Konzertiert mit der Ausbildung des Spalts findet in Schritt e. das Ausbringen des pharmazeutischen Fluids aus der Vorrichtung statt. Durch das Ausbringen erniedrigt sich der auf das pharmazeutische Fluid wirkende Druck.

Erreicht der auf das pharmazeutische Fluid wirkende Druck, bedingt durch fortgesetztes Ausbringen des Fluids, einen Wert unter oder gleich 5 N/cm², zieht sich der Schlauch radial zusammen, wobei der zuvor gebildete Spalt und die Vorrichtung am zweiten Schlauchende wieder fluidleitend geschlossen wird.

Wird weiterhin Druck auf das pharmazeutische Fluid aus Richtung des ersten Schlauchendes ausgeübt, kommt es bei einer weiteren Überschreitung des Drucks von 5 N/cm² zu einer weiteren Ausbringung des pharmazeutischen Fluids.

Mit dem beschriebenen Verfahren ist es möglich, kleine Volumina an pharmazeutischen Fluid, beispielsweise bis zu 2 Millilitern, kontrolliert und lokal abzugeben. Sobald der auf das pharmazeutische Fluid ausgeübt Druck unter die Schwelle von 5 N/cm² kommt, stoppt die Applikation innerhalb kürzester Zeit, beispielsweise innerhalb einer Sekunde.

Die für die Vorrichtung offenbarten Merkmale sind auch für das System und das Verfahren offenbart und umgekehrt.

### Figuren

Die Erfindung wird im Folgenden durch Figuren weiter beispielhaft illustriert. Die Erfindung ist nicht auf die Figuren beschränkt.

Es zeigen
- Fig. 1: einen teilweisen schematischen Längsschnitt einer Vorrichtung zum Applizieren eines pharmazeutischen Fluids umfassend einen Schlauch und ein Ventilelement,
- Fig. 2: die Vorrichtung aus Figur 1 in einem schematischen Längsschnitt durch das Ventilelement,
- Fig. 3: die Vorrichtung aus den Figuren 1 und 2 mit gefördertem pharmazeutischen Fluid,
- Fig. 4: die Vorrichtung aus den Figuren 1 bis 3 beim Applizieren des pharmazeutischen Fluids,
- Fig. 5: ein System zur Applikation eines pharmazeutischen Fluids, und
- Fig. 6: ein Verfahren zur Applikation eines pharmazeutischen Fluids.

### Beschreibung der Figuren

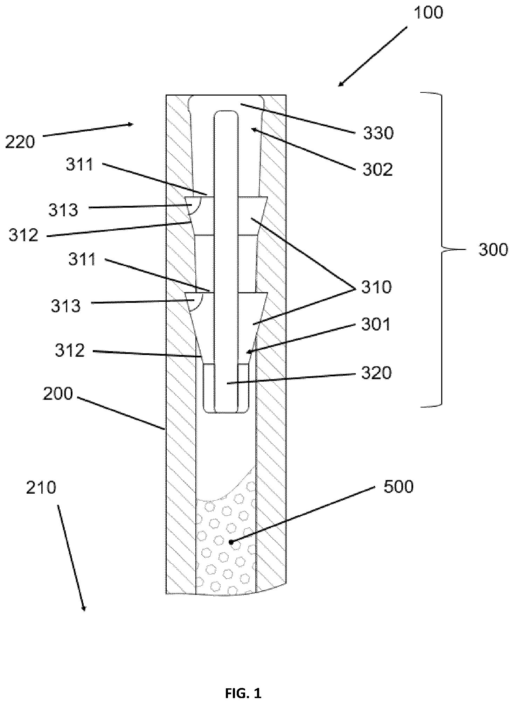

**Figur 1** zeigt einen teilweisen schematischen Längsschnitt einer beispielhaften Ausführung einer Vorrichtung 100 zum Applizieren eines pharmazeutischen Fluids 500. Die Vorrichtung 100 umfasst einen Schlauch 200 (nur abschnittsweise gezeigt) mit einem ersten Schlauchende 210 (nicht gezeigt) und einem dem ersten Schlauchende 210 entgegengesetztem zweiten Schlauchende 220. Figur 1 zeigt den Schlauch 200 in einem Längsschnitt.

Die Vorrichtung 100 umfasst weiterhin ein Ventilelement 300, gezeigt in einer Seitenansicht, welches innerhalb des Schlauchs 200, insbesondere innerhalb des zweiten Schlauchendes 220 angeordnet ist. Das Ventilelement weist an einem zweiten Ventilelementende 302 ein Dichtungselement 330 auf, welches das zweite Schlauchende 220 fluidleitend verschließt. Dazu weist das Dichtungselement 330 einen Dichtungselementdurchmesser auf, welcher größer (ungefähr 10 %) ist als ein Innendurchmesser des Schlauchs. In weiteren, nicht gezeigten Ausführungsformen der Vorrichtung 100 entspricht der Dichtungselementdurchmesser dem Innendurchmesser des Schlauchs.

Das Ventilelement 300 weist an einem ersten Ventilelementende 301 zwei hintereinander angeordnete Verankerungselemente 310 in Form konusförmiger Widerhakenelemente auf, welche mit einer Wandung des Schlauchs zusammenwirken und ein Austreiben des Ventilelements 300 aus dem zweiten Schlauchende 220 heraus unterbinden. Die Verankerungselemente 310 weisen dafür einen spitzen Winkel 313 zwischen einer Konusbasis 311 und einem Konusmantel 312 auf, so dass sich das Ventilelement 300 zwar einfach in das zweite Schlauchende 220 in der gezeigten Ausrichtung des Ventilelements 300 einschieben lässt, aber ein Ausschieben in die entgegengesetzte Richtung, also aus dem zweiten Schlauchende 220 heraus, durch die konusförmige Ausgestaltung der Verankerungselemente 310, welche in die flexible Wandung des Schlauchs 200 widerhakenähnlich eingreifen, unterbleibt. Um das Ventilelement 300 möglichst sicher gegen ein ungewolltes Austreiben aus dem zweiten Schlauchende 220 zu sichern, weist das Ventilelement 300 zwei hintereinanderliegende Verankerungselemente 310 auf. In weiteren, nicht gezeigten Ausführungsformen, weist das Ventilelement 300 lediglich ein Verankerungselement 310 oder drei und mehr hintereinanderliegende Verankerungselement 310 auf.

Damit das pharmazeutische Fluid 500 aus Richtung des ersten Schlauchendes 210 bis zum Dichtungselement 330 förderbar ist, umfasst das Ventilelement 300 vier Leitungsmittel 320, wobei lediglich eines in der gezeigten Seitenansicht zu sehen ist. Zwei weitere verlaufen in und ein weiteres hinter der Zeichenebene und sind somit in der Seitenansicht des Ventilelements 300 verdeckt. Die Leitungsmittel 320 durchbrechen die Verankerungselemente 310 in Form einer Nut und erstrecken sich entlang des ersten Ventilelementendes 301 bis zum Dichtungselement 330.

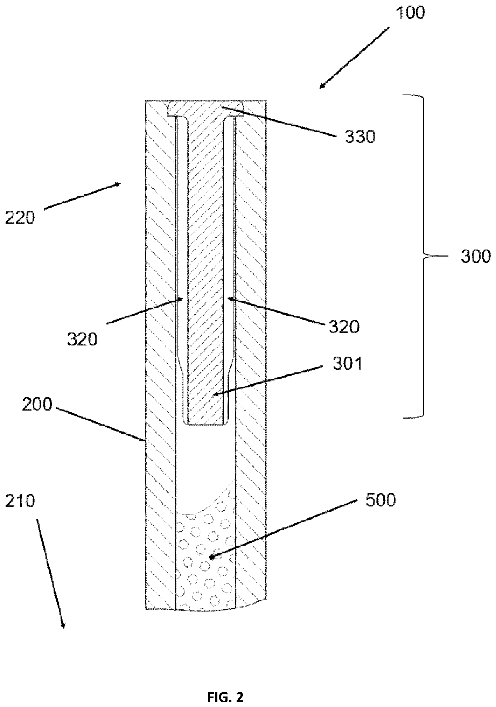

**Figur 2** zeigt die Vorrichtung 100 aus Figur 1, wobei im Gegensatz zu Figur 1 neben dem Schlauch 200 auch das Ventilelement 300 in einem Längsschnitt gezeigt ist. Die Verankerungsmittel 310 aus Figur 1 sind in der gezeigten Ansicht nicht sichtbar, wohingegen im Gegensatz zu Figur 1 die sich entlang der Zeichenebene erstreckenden Leitungsmittel 320 gezeigt sind. Die Leitungsmittel 320 erstrecken sich über das erste Ventilelementende 301 bis zum Dichtungselement 330.

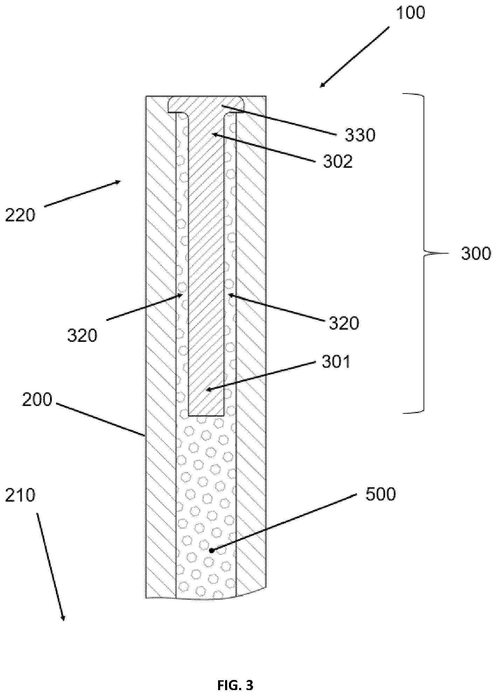

**Figur 3** zeigt die Vorrichtung 100 aus den Figuren 1 und 2, wobei das pharmazeutische Fluid 500 sich über die Leitungsmittel 320 bis zum Dichtungselement 330 erstreckt. Das Dichtungselement 330 verschließt das zweite Schlauchende 220 fluidleitend, so dass das pharmazeutische Fluid 500 nicht aus der Vorrichtung 100 ausfließen kann.

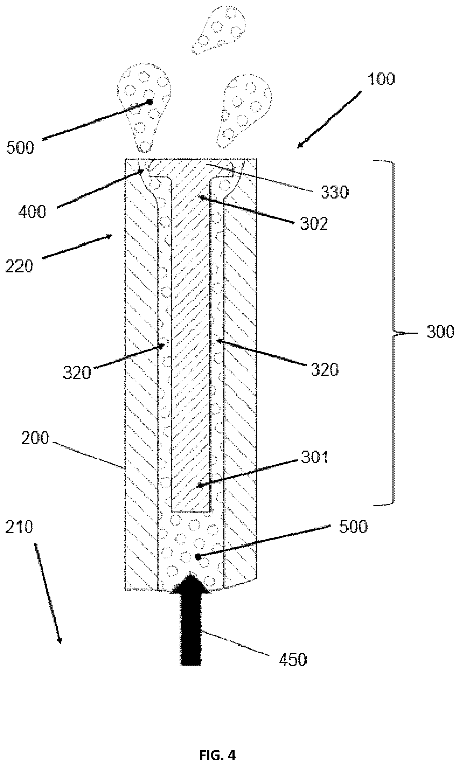

**Figur 4** zeigt die Vorrichtung 100 aus den Figuren 1 bis 3, wobei auf das pharmazeutische Fluid 500 aus Richtung des ersten Schlauchendes 210 eine Druckbeaufschlagung 450 (angedeutet durch einen Pfeil) ausgeübt wird. Die Druckbeaufschlagung 450 übersteigt einen Grenzwert von 5 N/cm², so dass sich der Schlauch 200 im Bereich des Dichtungselements 330 am zweiten Schlauchende 220 radial unter Ausbildung eines Spalts 400 dehnt. In weiteren Ausführungsformen liegt der Grenzwert der Druckbeaufschlagung 450 niedriger oder höher als 5 N/cm². Durch den Spalt 400 ist das zweite Schlauchende 220 fluidleitend geöffnet und das pharmazeutische Fluid 500 kann einer gewünschten Stelle, insbesondere in einem Patienten, appliziert werden. Der Spalt 400 bleibt solange bestehen, wie die Druckbeaufschlagung 450 den Grenzwert übersteigt. Unterschreitet die Druckbeaufschlagung 450 den Grenzwert, kommt es augenblicklich zu einer radialen Kontraktion des zweiten Schlauchendes 220 und der Schlauch 200 ist durch das Dichtungselement 330 wieder fluidleitend verschlossen.

Die **Figuren 1 bis 4** zeigen somit einen Appliziervorgang eines pharmazeutischen Fluids 500 mittels der Vorrichtung 100. Der Appliziervorgang ist wiederholbar.

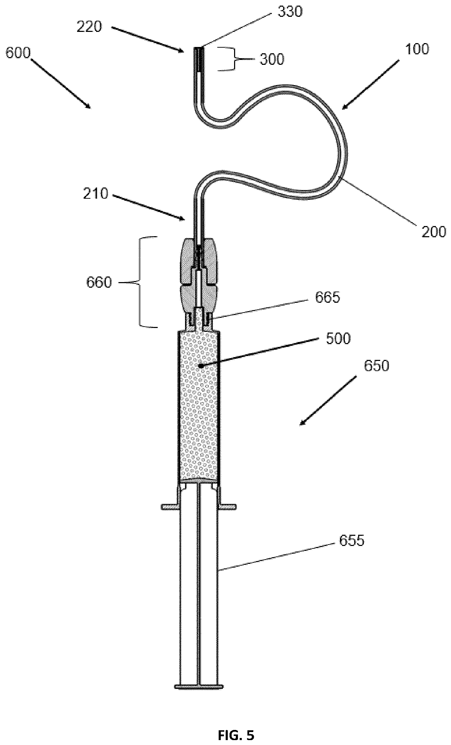

**Figur 5** zeigt ein System 600 zur Applikation eines pharmazeutischen Fluids 500 umfassend ein Reservoir 650 für das pharmazeutisches Fluid 500 in Form einer Spritze und die Vorrichtung 100 aus den Figuren 1 bis 4. Die Vorrichtung 100 ist über das erste Schlauchende 210 fluidleitend mit dem Reservoir 650 mittels eines Adapter 660 aufweisend einen Luer/Lock-Anschluss 665 verbunden. In dem gezeigten Zustand ist das zweite Schlauchende 220 fluidleitend durch das Ventilelement 300 verschlossen. Um das pharmazeutische Fluid 500 aus dem System 100 auszutragen, kann ein Fördermittel 655 in Form eines Kolbens axial in das Reservoir 650 eingeschoben werden, wodurch das pharmazeutische Fluid 500 über den Schlauch 200 bis zum Ventilelement 300, insbesondere dem Dichtungselement 330, gefördert wird. Erreicht die Druckbeaufschlagung den vorgesehenen Grenzwert, bildet sich ein Spalt (nicht gezeigt) zwischen Schlauch und Dichtungselement 330 aus, durch welchen das pharmazeutische Fluid 500 an die gewünschte Stelle appliziert werden kann.

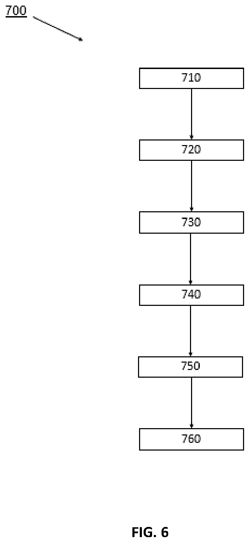

**Figur 6** zeigt ein Flussdiagram eines Verfahrens zur Applikation eines pharmazeutischen Fluids 500 mittels der Vorrichtung 100 umfassend die Schritte 710 bis 760. Die Vorrichtung 100 umfasst den Schlauch 200 und das Ventilelement 300,
wobei der Schlauch 200 über das erste Schlauchende 210 mit einem Reservoir für das pharmazeutische Fluid 500 fluidleitend verbindbar ist und wobei das Ventilelement 300 in dem zweiten Schlauchende 220 des Schlauchs 200 angeordnet ist,
wobei das Ventilelement 300
an dem dem ersten Schlauchende 210 zugewandten ersten Ventilelementende 301 mindestens ein Verankerungselement 310 aufweist, um ein Ausbringen des Ventilelements 300 aus dem zweiten Schlauchende 220 zu verhindern,
an dem dem zweiten Schlauchende 220 zugewandten zweiten Ventilelementende 302 das Dichtungselement 330 mit einem Dichtungselementaußendurchmesser aufweist, welcher zumindest einem Schlauchinnendurchmesser des Schlauchs 200 entspricht, so dass das zweite Schlauchende 220 durch das Dichtungselement 330 fluidleitend verschlossen ist, und das Leitungsmittel 320 umfasst, welches das erste Schlauchende 210 und das Dichtungselement 330 fluidleitend verbindet 1.

In Schritt 710 erfolgt ein fluidleitendes Verbinden der Vorrichtung 100 mit einem Reservoir für das pharmazeutische Fluids 500. Dabei kann das fluidleitende Verbinden 710 zu unterschiedlichen Zeitpunkten stattfinden. Beispielsweise kann der Schritt 710 erst kurz, beispielsweise 5 Minuten, vor einer Verwendung der Vorrichtung 100, bei der Herstellung der Vorrichtung 100 oder zu jedem Zeitpunkt zwischen diesen Zeitpunkten unkten stattfinden.

In Schritt 720 findet ein Fördern des pharmazeutischen Fluids 500 aus dem Reservoir über den Schlauch 200 bis zum Dichtungselement 330 statt. Ist das Reservoir beispielsweise eine Spritze 650, wie in Figur 5 dargestellt, kann Schritt 720 durch axiales Einschieben eines zu der Spritze 650 zugehörigen Kolbens 655 ausgeführt werden.

Das Ventilelement 300 verschließt das zweite Schlauchende 220 solange, bis in Schritt 730 ein Druck von mindestens 5 N/cm² aus Richtung des ersten Schlauchendes 210 auf das pharmazeutische Fluid 500 ausgeübt wird.

Bei Erreichung dieses Grenzwertes von 5 N/cm² kommt es in Schritt 740 zu einer radialen Dehnung des Schlauchs 200 im Bereich des Dichtungselements 330 unter Ausbildung eines Spalts 400 zwischen Dichtungselement 330 und Schlauch 200. Dadurch ist das zweite Schlauchende 220 nicht mehr fluidleitend durch das Dichtungselement 330 verschlossen. Vorzugsweise erfolgt das Fördern in Schritt 720 und der Aufbau des Drucks zur Ausbildung des Spalts 400 in Schritt 740 mit den gleichen Mitteln, beispielsweise durch axiales Einschieben eines Kolbens statt.

Mit Ausbildung des Spalts 400 in Schritt 740 erfolgt in Schritt 750 ein Ausbringen des pharmazeutischen Fluids 500 durch den Spalt 400 aus der Vorrichtung 100. Durch das Ausfließen des pharmazeutischen Fluids 500 aus der Vorrichtung 100 erfolgt ein Druckabbau innerhalb der Vorrichtung 100. Fällt der Druck durch das Ausbringen des Fluids 500 unter den Grenzwert von 5 N/cm² kommt es in Schritt 760 zu einem radialen Zusammenziehen des Schlauchs, wodurch sich der zuvor gebildete Spalt 400 zwischen Dichtungselement 300 und Schlauch 200 wieder schließt und das zweite Schlauchende 220 fluiddicht verschlossen wird. Die Vorrichtung 100 schließt sich somit durch das Ausbringen des pharmazeutischen Fluids 500 in Schritt 750 wieder selbstständig, sofern nicht durch fortgeführte Druckausübung auf das pharmazeutische Fluid 500 ein Druck von mindestens 5 N/cm² trotz des Ausbringens aufrechterhalten wird. Die Vorrichtung 100 eignet sich daher insbesondere zum kontrollierten Applizieren einer kleinvolumigen Menge, beispielsweise bis zu 2 Millilitern, an pharmazeutischen Fluids 500. Das Verfahren kann beliebig häufig wiederholt werden.

### Bezugszeichen

- 100: Vorrichtung
- 200: Schlauch
- 210: erstes Schlauchende
- 220: zweites Schlauchende
- 300: Ventilelement
- 301: erstes Ventilelementende
- 302: zweites Ventilelementende
- 310: Verankerungselement
- 311: Konusbasis
- 312: Konusmantel
- 313: Winkel zwischen Konusbasis und Konusmantel
- 320: Leitungsmittel
- 330: Dichtungselement
- 400: Spalt
- 450: Druckbeaufschlagung
- 500: pharmazeutisches Fluid
- 600: System
- 650: Reservoir
- 655: Fördermittel
- 660: Adapter
- 665: Luer/Lock - Anschluss
- 700: Verfahren
- 710: fluidleitendes Verbinden
- 720: Fördern
- 730: Druckaufbau
- 740: radiale Dehnung
- 750: Ausbringen
- 760: radiales Zusammenziehen

## Patentansprüche

1. Vorrichtung (100) zur Applikation eines pharmazeutischen Fluids (500) umfassend einen Schlauch (200) und ein Ventilelement (300),
wobei der Schlauch (200) über ein erstes Schlauchende (210) mit einem Reservoir (650) für das pharmazeutische Fluid (500) fluidleitend verbindbar ist und wobei das Ventilelement (300) in einem zweiten Schlauchende (220) des Schlauchs (200) angeordnet ist,
**dadurch gekennzeichnet, dass** das Ventilelement (300)
an einem dem ersten Schlauchende (210) zugewandten ersten Ventilelementende (301) mindestens ein Verankerungselement (310) aufweist, um ein Ausbringen des Ventilelements (300) aus dem zweiten Schlauchende (220) zu verhindern,
an einem dem zweiten Schlauchende (220) zugewandten zweiten Ventilelementende (302) ein Dichtungselement (330) mit einem Dichtungselementaußendurchmesser aufweist, welcher zumindest einem Schlauchinnendurchmesser des Schlauchs (200) entspricht, so dass das zweite Schlauchende (220) durch das Dichtungselement (310) fluidleitend verschlossen ist, und
ein Leitungsmittel (320) umfasst, welches das erste Schlauchende (210) und das Dichtungselement (330) fluidleitend verbindet,
wobei bei einer Druckbeaufschlagung (450) auf das pharmazeutische Fluid (500) aus Richtung des ersten Schlauchendes (210) reversibel ein Spalt (400) zwischen dem Schlauch (200) und dem Dichtungselement (330) ausformbar ist, so dass das zweite Schlauchende (220) fluidleitend geöffnet ist.

2. Vorrichtung (100) nach Anspruch 1, wobei bei einer Druckbeaufschlagung (450) von mindestens 5 N/cm² auf das pharmazeutische Fluid (500) aus Richtung des ersten Schlauchendes (210) reversibel ein Spalt (400) zwischen dem Schlauch (200) und dem Dichtungselement (330) ausformbar ist, so dass das zweite Schlauchende (220) fluidleitend geöffnet ist.

3. Vorrichtung (100) nach einem der Ansprüche 1 oder 2, wobei das Ventilelement (300) eine Ventilelementlänge aufweist, welche mindestens dreimal dem Dichtungselementaußendurchmesser entspricht.

4. Vorrichtung (100) nach einem der vorhergehenden Ansprüche, wobei das mindestens eine Verankerungselement (310) mindestens ein Widerhakenelement ist.

5. Vorrichtung (100) nach Anspruch 4, wobei das mindestens eine Widerhakenelement als Konus mit einer Konusbasis (311) und einem Konusmantel (312) ausgeformt ist, wobei der Konusmantel (312) dem ersten Ventilelementende (301) und die Konusbasis (311) dem zweiten Ventilelementende (302) zugewandt ist.

6. Vorrichtung (100) nach Anspruch 5, wobei die Konusbasis (311) und der Konusmantel (312) einen Winkel (313) von maximal 80° einschließen.

7. Vorrichtung (100) nach einem der Ansprüche 5 oder 6, wobei der Konus zumindest an einer Stelle durch das Leitungsmittel (320) durchbrochen ist.

8. Vorrichtung (100) nach einem der vorhergehenden Ansprüche, wobei der Schlauch (200) ein elastisches Polymer mit einer Shore A-Härte von größer 50 und/oder ein thermoplastisches Polymer mit einer Shore D-Härte von größer 10 umfasst.

9. Vorrichtung (100) nach einem der vorhergehenden Ansprüche, wobei der Schlauch (200) einen Röntgenopaker aufweist.

10. Vorrichtung (100) nach einem der vorhergehenden Ansprüche, wobei innerhalb des Schlauchs (200) und/oder in einer Wandung des Schlauchs (200) mindestens eine Metallspirale entlang einer Längsachse der Vorrichtung (100) angeordnet ist.

11. Vorrichtung (100) nach einem der vorhergehenden Ansprüche, wobei die Vorrichtung (100) einen Adapter (660) aufweist, mit dem die Vorrichtung (100) und ein Reservoir (650) für ein pharmazeutisches Fluid (500) fluidleitend, insbesondere reversibel fluidleitend, verbindbar sind.

12. Vorrichtung (100) nach einem der vorhergehenden Ansprüche, wobei die Vorrichtung (100) innerhalb des Schlauchs (200) ein Rückschlagventil aufweist, welches in Richtung des ersten Schlauchendes (210) fluidundurchlässig ausgestaltet ist.

13. System (600) zur Applikation eines pharmazeutischen Fluids (500) umfassend ein Reservoir (650) für das pharmazeutisches Fluid (500) und eine Vorrichtung (100) nach einem der vorhergehenden Ansprüche 1 bis 12.

14. System (600) nach Anspruch 13, wobei das Reservoir (650) ein Fördermittel (655) zum Fördern eines pharmazeutischen Fluids (500) aus dem Reservoir (650) in das erste Schlauchende (210) und zum Erzeugen der Druckbeaufschlagung (450) auf das pharmazeutische Fluid (500) aus Richtung des ersten Schlauchendes (210) umfasst.

## Claims

1. A device (100) for applying a pharmaceutical fluid (500), comprising a tube (200) and a valve element (300),
the tube (200) being fluidically connectable to a reservoir (650) for the pharmaceutical fluid (500) via a first tube end (210), and the valve element (300) being arranged in a second tube end (220) of the tube (200),
**characterized in that** the valve element (300)
comprises at least one anchoring element (310) at a first valve element end (301) facing the first tube end (210), in order to prevent the valve element (300) from being discharged from the second tube end (220),
comprises a sealing element (330) at a second valve element end (302) facing the second tube end (220), the sealing element having a sealing element outer diameter corresponding to at least one inner tube diameter of the tube (200), so that the second tube end (220) is fluidically closed by the sealing element (310), and
comprises a duct means (320) which fluidically connects the first tube end (210) and the sealing element (330),
it being possible to reversibly form a gap (400) between the tube (200) and the sealing element (330) when pressure is applied (450) to the pharmaceutical fluid (500) from the direction of the first tube end (210), so that the second tube end (220) is fluidically open.

2. The device (100) according to claim 1, wherein a gap (400) can be reversibly formed between the tube (200) and the sealing element (330) when a pressure of at least 5 N/cm² is applied (450) to the pharmaceutical fluid (500) from the direction of the first tube end (210), so that the second tube end (220) is fluidically open.

3. The device (100) according to either claim 1 or claim 2, wherein the valve element (300) has a valve element length corresponding to at least three times the sealing element outer diameter.

4. The device (100) according to any of the preceding claims, wherein the at least one anchoring element (310) is at least one barb element.

5. The device (100) according to claim 4, wherein the at least one barb element is formed as a cone having a cone base (311) and a cone shell (312), wherein the cone shell (312) faces the first valve element end (301) and the cone base (311) faces the second valve element end (302).

6. The device (100) according to claim 5, wherein the cone base (311) and the cone shell (312) include an angle (313) of at most 80°.

7. The device (100) according to either claim 5 or claim 6, wherein the duct means (320) passes through the cone at least at one location.

8. The device (100) according to any of the preceding claims, wherein the tube (200) comprises an elastic polymer having a Shore A hardness of greater than 50 and/or a thermoplastic polymer having a Shore D hardness of greater than 10.

9. The device (100) according to any of the preceding claims, wherein the tube (200) comprises an x-ray opacifier.

10. The device (100) according to any of the preceding claims, wherein at least one metal coil is arranged along a longitudinal axis of the device (100) within the tube (200) and/or in a wall of the tube (200).

11. The device (100) according to any of the preceding claims, wherein the device (100) comprises an adapter (660) to which the device (100) and a reservoir (650) for a pharmaceutical fluid (500) can be fluidically, in particular reversibly fluidically, connected.

12. The device (100) according to any of the preceding claims, wherein the device (100) comprises a non-return valve within the tube (200), which non-return valve is designed to be fluid-impermeable in the direction of the first tube end (210).

13. A system (600) for applying a pharmaceutical fluid (500), comprising a reservoir (650) for the pharmaceutical fluid (500) and a device (100) according to any of the preceding claims 1 to 12.

14. The system (600) according to claim 13, wherein the reservoir (650) comprises a conveying means (655) for conveying a pharmaceutical fluid (500) from the reservoir (650) into the first tube end (210) and for generating the pressure application (450) on the pharmaceutical fluid (500) from the direction of the first tube end (210).

## Revendications

1. Dispositif (100) d'application d'un fluide pharmaceutique (500), comprenant un tuyau (200) et un élément de soupape (300),
le tuyau (200) pouvant être raccordé en communication fluidique, par une première extrémité de tuyau (210), avec un réservoir (650) pour le fluide pharmaceutique (500) et l'élément de soupape (300) étant agencé dans une seconde extrémité de tuyau (220) du tuyau (200), **caractérisé en ce que** l'élément de soupape (300)
présente au moins un élément d'ancrage (310) sur une première extrémité d'élément de soupape (301) orientée vers la première extrémité de tuyau (210), pour empêcher un retrait de l'élément de soupape (300) de la seconde extrémité de tuyau (220),
présente un élément d'étanchéité (330), ayant un diamètre extérieur d'élément d'étanchéité, sur une seconde extrémité d'élément de soupape (302) orientée vers la seconde extrémité de tuyau (220), lequel diamètre correspond au moins à un diamètre intérieur de tuyau du tuyau (200), de sorte que la seconde extrémité de tuyau (220) soit fermée en communication fluidique par l'élément d'étanchéité (310) et
comprend un moyen formant conduit (320), lequel relie en communication fluidique la première extrémité de tuyau (210) et l'élément d'étanchéité (330),
une fente (400) pouvant se former de manière réversible entre le tuyau (200) et l'élément d'étanchéité (330) lorsqu'une pression (450) est exercée sur le fluide pharmaceutique (500) venant de la direction de la première extrémité de tuyau (210), de sorte que la seconde extrémité de tuyau (220) soit ouverte en communication fluidique.

2. Dispositif (100) selon la revendication 1, dans lequel, lorsqu'une pression (450) d'au moins 5 N/cm² est exercée sur le fluide pharmaceutique (500) venant de la direction de la première extrémité de tuyau (210), une fente (400) peut se former de manière réversible entre le tuyau (200) et l'élément d'étanchéité (330), de sorte que la seconde extrémité de tuyau (220) soit ouverte en communication fluidique.

3. Dispositif (100) selon l'une quelconque des revendications 1 ou 2, dans lequel l'élément de soupape (300) présente une longueur d'élément de soupape, laquelle correspond à au moins trois fois le diamètre extérieur d'élément d'étanchéité.

4. Dispositif (100) selon l'une quelconque des revendications précédentes, dans lequel l'au moins un élément d'ancrage (310) est au moins un élément formant loquet.

5. Dispositif (100) selon la revendication 4, dans lequel l'au moins un élément formant loquet est réalisé sous forme de cône comportant une base de cône (311) et une enveloppe de cône (312), l'enveloppe de cône (312) étant orientée vers la première extrémité d'élément de soupape (301) et la base de cône (311) étant orientée vers la seconde extrémité d'élément de soupape (302).

6. Dispositif (100) selon la revendication 5, dans lequel la base de cône (311) et l'enveloppe de cône (312) forment un angle (313) maximal de 80°.

7. Dispositif (100) selon l'une quelconque des revendications 5 ou 6, dans lequel le moyen formant conduit (320) traverse le cône à au moins un endroit.

8. Dispositif (100) selon l'une quelconque des revendications précédentes, dans lequel le tuyau (200) comprend un polymère élastique ayant une dureté Shore A supérieure à 50 et/ou un polymère thermoplastique ayant une dureté Shore D supérieure à 10.

9. Dispositif (100) selon l'une quelconque des revendications précédentes, dans lequel le tuyau (200) présente un radio-opaque.

10. Dispositif (100) selon l'une quelconque des revendications précédentes, dans lequel, à l'intérieur du tuyau (200) et/ou dans une paroi du tuyau (200), au moins une spirale métallique est agencée le long d'un axe longitudinal du dispositif (100).

11. Dispositif (100) selon l'une quelconque des revendications précédentes, le dispositif (100) comportant un adaptateur (660) à l'aide duquel le dispositif (100) et un réservoir (650) pour un fluide pharmaceutique (500) peuvent être reliés en communication fluidique, en particulier en communication fluidique de manière réversible.

12. Dispositif (100) selon l'une quelconque des revendications précédentes, le dispositif (100) comportant, à l'intérieur du tuyau (200), une soupape de retenue réalisée de façon imperméable aux fluides dans la direction de la première extrémité de tuyau (210).

13. Système (600) d'application d'un fluide pharmaceutique (500), comprenant un réservoir (650) pour le fluide pharmaceutique (500) et un dispositif (100) selon l'une quelconque des revendications 1 à 12 précédentes.

14. Système (600) selon la revendication 13, dans lequel le réservoir (650) comprend un moyen de transport (655) pour le transport d'un fluide pharmaceutique (500) du réservoir (650) vers la première extrémité de tuyau (210) et pour la génération de la pression (450) exercée sur le fluide pharmaceutique (500) venant de la direction de la première extrémité de tuyau (210).
